(19) Europäisches Patentamt
European Patent Office
Office européen des brevets

(11) Veröffentlichungsnummer: **0 495 349 B1**

(12) **EUROPÄISCHE PATENTSCHRIFT**

(45) Veröffentlichungstag der Patentschrift: **23.08.95**

(51) Int. Cl.6: **A23K 1/16**, A23K 1/18, A61K 9/52

(21) Anmeldenummer: **92100001.4**

(22) Anmeldetag: **01.01.92**

(54) **Wirkstoffzubereitung zur oralen Verabreichung insbesondere an Wiederkäuer.**

(30) Priorität: **15.01.91 DE 4100920**

(43) Veröffentlichungstag der Anmeldung:
**22.07.92 Patentblatt 92/30**

(45) Bekanntmachung des Hinweises auf die
Patenterteilung:
**23.08.95 Patentblatt 95/34**

(84) Benannte Vertragsstaaten:
**AT BE CH DE DK ES FR GB GR IT LI LU NL PT SE**

(56) Entgegenhaltungen:
EP-A- 0 218 928    EP-A- 0 236 002
EP-A- 0 268 533    WO-A-87/03172
DE-B- 1 127 542    GB-A- 2 020 181
NL-C- 57 258       US-A- 3 166 476

(73) Patentinhaber: **Degussa Aktiengesellschaft**
**Weissfrauenstrasse 9**
**D-60311 Frankturt (DE)**

(72) Erfinder: **Greissinger, Dieter, Dr.**
**Kartäuserstrasse 2**
**W-6380 Bad Homburg (DE)**
Erfinder: **Kniesel, Heidemarie, Dr.**
**Franziskusstrasse 4**
**W-8759 Hösbach (DE)**
Erfinder: **Heimbeck, Winfried, Dr.**
**Breslauer Strasse 23a**
**W-8752 Mömbris (DE)**
Erfinder: **Tanner, Herbert, Dr.**
**Wildaustrasse 20**
**W-6450 Hanau 9 (DE)**

**Beschreibung**

Die Erfindung betrifft eine Wirkstoffzubereitung zur oralen Verabreichung insbesondere an Wiederkäuer, enthaltend einen mindestens eine biologisch wirksame Substanz enthaltenden Wirkstoffkern und eine diesen Kern umhüllende Beschichtung, die eine verzögerte Freisetzung des Kerns nach der oralen Verabreichung bewirkt, sowie die Verwendung dieser Wirkstoffzubereitung zur Ernährung oder veterinärmedizinischen Versorgung von Wiederkäuern und ein Verfahren zur Versorgung von Wiederkäuern mit Wirkstoffen.

Tieren, die zur Produktion von beispielsweise Fleisch, Milch, Wolle oder Eiern aufgezogen und gehalten werden, müssen häufig zusätzliche Wirkstoffe verabreicht werden, z.B. zur Versorgung mit den je nach Futterzusammensetzung limitierenden Aminosäuren oder mit Vitaminen oder mit veterinärmedizinisch wirksamen Substanzen. Bei Monogastern ist dies im allgemeinen problemlos, weil ihnen die zuzuführenden Wirkstoffe ganz einfach oral verabreicht werden können, z.B. als Zusatz eingemischt in das Futter oder in Form von mit dem Wirkstoff gefüllten Gelatinekapseln. Bei Wiederkäuern aber versagen derlei einfache Methoden in der Regel, weil die zuzuführenden Wirkstoffe bereits im Pansen ganz oder zumindest größtenteils durch die Pansenflora verstoffwechselt werden und allenfalls in geringer Menge im anschließenden Intestinaltrakt für die direkte Resorption zur Verfügung stehen. Wenn noch dazu eine verzögerte Freisetzung des Wirkstoffes gewünscht ist, dann bereitet dies auch bei Monogastern, insbesondere bei großen Mengen, Schwierigkeiten, da der Wirkstoff in der Regel entweder an eine Matrix gebunden oder in eine solche eingebettet wird und die Matrix meist nur mit wenig Wirkstoff beladen werden kann. Auch diffusionskontrollierte Wirkstofffreigaben sind diffizil in der Abstimmung und kaum geeignet für größere Mengen. Allen gemeinsam sind die hohen Präparatekosten.

Zur Lösung dieser Probleme sind bereits beinahe unzählige Vorschläge bekannt. So beschreibt die DE-A-11 27 542 die Herstellung von Pillen zur Verabfolgung biologisch aktiver Substanzen an Wiederkäuer, wobei die Pille eine Dichte und ein Gewicht hat, welche diese relativ beständig in dem Wiederkäuernetzmagen des Tieres nach Verabfolgung und Einlagerung in dem Magen zurückhält, und wobei der Pille eine biologisch aktive Substanz einverleibt wird, welche von der Pille während einer ausgedehnten Zeitdauer in den Inhalt des Magens in Freiheit gesetzt wird. Das Verfahren der DE-A-11 27 542 kennzeichnet sich dadurch, daß auf einen Kern, welcher die biologisch aktive Substanz enthält, durch die an sich bekannte Technik der Druckumhüllung, eine Schicht hoher Dichte gepreßt wird, in welcher man ein Loch freiläßt, welches bis an den Kern heran oder in den Kern hineinreicht.

Aus der WO 87/03172 schließlich sind Kapseln für die orale Verabreichung an Wiederkäuer bekannt, die einen Wirkstoffkern aufweisen, welcher von einer Hülle umgeben ist, die aus einer physiologisch verträglichen Beschichtung besteht. Diese weist wenigstens einen Abschnitt auf, welcher aus einem anderen Material als die restliche Beschichtung besteht, wobei dieses andere Material sich nach Durchgang durch den Pansen innerhalb von sechs Stunden auflöst.

Allen bekannten Vorschlägen ist gemeinsam, daß die zuzuführenden Wirkstoffe durch Einbetten in eine Matrix oder durch Umhüllen mit einer Beschichtung gegen vorzeitige Freisetzung im Pansen oder Magen geschützt werden. Für die Matrix beziehungsweise die Beschichtung werden die verschiedenartigsten Materialien verwendet. Keiner der bekannten Lösungsvorschlage ist jedoch voll befriedigend, weil als Komponenten für die Schutzmaterialien Stoffe verwendet werden müssen, die entweder überhaupt nicht oder zumindest bisher noch nicht nahrungs- oder futtermittelrechtlich zugelassen sind, oder nicht ohne weiteres verfügbar sind, einer aufwendigen Vorbehandlung bedürfen oder dazu führen, daß mit ihrer Hilfe hergestellte Teilchen bei der Lagerung zum Verkleben neigen und/oder thermisch und mechanisch nicht ausreichend widerstandsfähig sind.

Aufgabe der Erfindung ist daher, eine Wirkstoffzubereitung zur Verfügung zu stellen, die einen slow-release-Effekt oder einen weitgehenden Schutz des Wirkstoffes im Pansen oder Magen bietet, den Wirkstoff aber im sich anschließenden Intestinaltrakt freigibt, einfach und kostengünstig aus möglichst wenigen, physiologisch völlig unbedenklichen Stoffen hergestellt werden kann und thermisch und mechanisch belastbar ist. Aufgabe ist außerdem die Verwendung der Wirkstoffzubereitung und ein Verfahren zur Versorgung von Wiederkäuern mit einem Wirkstoff.

Diese Aufgabe wird hinsichtlich der Wirkstoffzubereitung bei einer Wirkstoffzubereitung der eingangs beschriebenen Art dadurch gelöst, daß die umhüllende Beschichtung dünnere Bereiche und Sollbruchstellen aufweist, die die verzögerte Freisetzung gegenüber einer im wesentlichen gleichmäßigen Beschichtung beschleunigen.

Unter Sollbruchstellen werden solche Bereiche verstanden, die zur Ablösung ganzer Beschichtungteile als solches oder zum Auseinanderbrechen der Wirkstoffzubereitung führt. Dies kann z. B. dadurch geschehen, daß eine im wesentlichen kapselförmige Wirkstoffzubereitung in der Mitte auseinanderbricht, oder daß sich einer oder mehrere Deckel z. B. eines Pellets ablösen. Üblicherweise sind die Sollbruchstellen Verjüngungen oder dünnere Bereiche, vorzugsweise in Form eines Ringes. Die umhüllende Beschichtung sollte aus einem Material bestehen, das bei der Körpertemperatur des Säugers, dem die Wirkstoffzubereitung verabreicht wird, nicht schmierend, sondern vorzugsweise spröde ist. Eine schmierende Hülle würde die in die Beschichtung eingebrachten Sollbruchstellen infolge der Magen- oder Darmtätigkeit wieder zudecken, so daß der gewünschte Effekt nicht eintreten kann. Ebenso sind weiche bzw. zu elastische Umhüllungen wenig geeignet, da diese infolge ihrer stoßabsorbierenden Eigenschaft nur schwer aufbrechen und den Wirkstoff freigeben. Andererseits sollte die Umhüllung so stabil sein, daß die Wirkstoffzubereitung beim gewöhnlichen Handling nicht schon auseinanderbricht. Das kann z. B. auch dadurch erreicht werden, daß der Wirkstoffkern nicht ein loses Pulver, sondern insgesamt fest ist, so daß kleinere Beschädigungen der Beschichtung schon vor der oralen Verabreichung unschädlich sind, da vor der Verabreichung kein Wirkstoff durch die Beschädigung verloren geht und nach der oralen Verabreichung nur eine kleine Öffnung besteht, aus der der Wirkstoff nur langsam herausgelöst wird. Auch hierbei wird eine genügende Verzögerung der Freisetzung des Wirkstoffes erzielt.

Die Beschichtung wird im Regelfall aus einem Filmbildner aufgebaut, wobei als Filmbildner natürlich vorkommende oder modifizierte natürlich vorkommende Polymere oder Homo- und Copolymere, die nach üblichen bekannten Verfahren herstellbar sind, eingesetzt werden können. Solche geeigneten Polymere sind beispielsweise Cellulose-ester, -ether, -acylate, Poly(meth)acrylate, Polyamide, Polyester bzw. Copolymere aus z. B. Acrylnitril, einem gegebenenfalls substituiertem Vinylpyridin mit beispielsweise Styrol, Ethylen, Propylen, Butadien oder Estern und Amiden der Methacrylsäure oder Acrylsäure. Gegebenenfalls kann dem Filmbildner ein Weichmacher wie z. B. Diethylphthalat, Polyethylenglycol oder ein Citronensäureester oder weitere Hilfsstoffe wie Kieselsäure, Talkum oder Alkali- oder Erdalkalistearate zugesetzt werden. Die Beschichtung soll so gewählt sein, daß keine zu schnelle Auflösung erfolgt. Erfindungsgemäß können sogar Filmbildner eingesetzt werden, die bei gleichmäßiger Beschichtung keine oder nur sehr geringe (< 50 %) Freisetzung des Wirkstoffes erlauben, durch die Sollbruchstellen erfolgt dennoch die gewünschte Wirkung.

Ganz besonders geeignet ist eine Beschichtung auf Basis eines Celluloseethers, vorzugsweise Ethylcellulose und insbesondere eine solche Beschichtung mit einer zusätzlichen inneren gefüllten Schicht.

Vorzugsweise macht die Beschichtung 1,5 bis 30 Gew.-%, bezogen auf das Gewicht des Wirkstoffkerns, aus. Vorteilhaft ist hierbei, wenn der Filmbildner, wiederum bezogen auf das Gewicht des Wirkstoffkerns, zu 1 bis 20 Gew.-% und der Hilfs- oder Füllstoff zu 0.5 bis 10 Gew.-% vorliegt, wobei die Kombination aus beidem am günstigsten ist. Hierbei sollte üblicherweise das Verhältnis zwischen Filmbildner und Füllstoff im Bereich 10 : 1 bis 1 : 5 betragen, bevorzugt ist ein Verhältnis zwischen 5 : 1 bis 1 : 2.

Geeignete Füllstoffe sind insbesondere Metallcarbonate, Kieselsäuren, Silikate, Alginate, Stearate, Stärken und Gummis. Solche geeigneten Füllstoffe sind z. B. Magnesium-, Calzium-, oder Natriumcarbonat, Fällungskieselsäure, Calzium-, Aluminium- oder Natriumaluminiumsilikat, Calzium-, Natrium- oder Aluminiumalginat, Natriumstearat, Maisstärke oder Gummi arabicum oder ein Gemisch aus zwei oder mehr dieser Stoffe. Die Partikelgröße der Füllstoffe ist von geringer Bedeutung, die Größe soll möglichst deutlich kleiner sein als die Schichtdicke und liegt üblicherweise im Bereich von 0,1 - 30 $\mu$m, bevorzugt 0,7 - 10 $\mu$m.

Besonders geeignete Beschichtungen enthalten zwei Schichten, eine innere aus der Gesamtmenge des Füllstoffs und 0,2 bis 8 Gew.-% Filmbildner, wiederum bezogen auf das Gewicht des Wirkstoffkerns, und einer äußeren Schicht, die den Rest an vorzugsweise dem gleichen Filmbildner enthält.

Eine solche Beschichtung ist genügend spröde, so daß sie nach und nach nach der oralen Verabreichung aufbricht, wodurch der Wirkstoff verzögert nach und nach freigesetzt wird. Die Doppelbeschichtung auf Basis eines Celluloseethers ist dabei auch geeignet, ohne die beschriebenen Sollbruchstellen aufzubrechen und den Wirkstoff freizugeben. Günstig sind hierbei auch die einfache Herstellung, die geringen Materialkosten und die nahrungs- bzw. futterrechtliche Zulassung. Besonders günstig ist die Kombination dieser Beschichtung mit den Sollbruchstellen, die das Aufbrechen begünstigen.

Die dünneren Bereiche, die auch zusammenhängend als ein Bereich z. B. flächenförmig oder vorzugsweise in Form von geschlossenen Linien, insbesondere kreisförmigen Linien vorliegen können, erstrecken sich günstigerweise über mind. 0,5 % (Flächenprozent) der Gesamtfläche der Beschichtung, in der Regel über mind. 1 %. Besonders geeignet sind dünnere Bereiche, die mind. 2 % der Gesamtfläche der Beschichtung ausmachen und insbesondere bei flächen- statt linienförmigen dünneren Bereichen liegt deren Fläche bei mind. 5 % der Gesamtfläche. Üblicherweise sollten die Flächen der dünneren Bereiche 20 % der Gesamtfläche der Beschichtung nicht überschreiten, insbesondere bei linienförmigen dünneren Bereichen werden in der Regel 10 % der Gesamtfläche nicht überschritten. Der dünnere Bereich sollte dabei mind. 20 % unter der mittleren Schichtdicke liegen, bevorzugt sind mind. 30 % und insbesondere mind. 50 %. Zu dünn sollte der dünnere Bereich wiederum auch nicht sein, da sonst zu schnell ein Bruch

EP 0 495 349 B1

eintreten kann. Günstig ist in der Regel, wenn mind. 10 % der mittleren Schichtdicke eingehalten werden. Grundsätzlich sollten kleinflächige dünnere Bereiche in der Schichtdicke besonders dünn sein, um sicher aufzubrechen.

Vorzugsweise werden die dünneren Bereiche und Sollbruchstellen durch Kanten gebildet. Solche Kanten treten beispielsweise bei Pellets oder Tabletten auf, und die dünneren Bereiche und/oder Sollbruchstellen werden leicht dadurch erreicht, daß die Kanten vor der Beschichtung nicht entschärft werden, sondern scharfkantig bleiben. Die Beschichtung erfolgt dann auf den Flächen dicker als auf den Kanten. Ebenso darf die gesamte Beschichtung nicht zu dick aufgetragen werden, da sonst trotz gebildeter dünnerer Bereiche die Freisetzung des Wirkstoffkerns zu langsam oder überhaupt nicht erfolgt. Als günstige mittlere Schichtdicke der Beschichtung wurden 5 bis 150 $\mu$m, insbesondere 10 bis 80 $\mu$m und besonders bevorzugt 20 bis 60 $\mu$m gefunden. Wenn die dünneren Bereiche und/oder Sollbruchstellen durch Kanten gebildet werden, dann schließen die angrenzenden Flächen, die die Kanten bilden, vorzugsweise einen Winkel von ≤ 120° ein, bevorzugt ≤ 90° und insbesondere ≤ 70°. Ein Winkel unter 20° sollte nicht unterschritten werden, bevorzugt sind Winkel über 45°. Vorzugsweise sind die an die Kante angrenzenden Flächen nicht zu klein, d. h. sie sollten eine Länge senkrecht zur Kante von mind. 0,05 mm, vorzugsweise mind. 0,1 mm und insbesondere mind. 0,2 mm haben. Hierbei ist günstig, wenn die Kanten einen nicht zu großen Radius aufweisen, d. h. sie sollten genügend scharfkantig sein. Geeignet ist ein Kantenradius von ≤ 1 mm, vorzugsweise ≤ 0,7 mm und insbesondere ≤ 0,5 mm. Besonders günstig ist hierbei, wenn der Radius der Kante höchstens die Länge der an die Kante angrenzenden Flächen senkrecht zur Kante hat, insbesondere sollte der Radius höchstens 2/3 und besonders günstig höchstens die Hälfte dieser Länge betragen. Solche Kanten, insbesondere die mit sehr kleinem Radius, sind genügend anfällig gegen mechanische Einwirkungen und besonders dünn beschichtet, so daß die Beschichtung an diesen Kanten aufbricht und nach und nach der Wirkstoff freigegeben wird.

Die sukzessive Freigabe des Wirkstoffes sollte durch Wahl der Beschichtung und Aufbau der Beschichtung für Wiederkäuer so bemessen sein, daß nach 6 h (≙ der durchschnittlichen Verweilzeit im Pansen) nach der oralen Verabreichung höchstens 50 %, vorzugsweise höchstens 30 % und insbesondere höchstens 20 % freigesetzt sind. Hingegen soll nach 24 h nach der oralen Verabreichung mind. 50 Gew.-%, vorzugsweise mind. 70 Gew.-% und insbesondere mind. 80 Gew.-% der biologisch wirksamen Substanz freigesetzt sein. Günstig ist dabei, wenn auch schon vor 6 h ein Teil der Wirkstoffe freigegeben wurde, da dies z. B. der Ernährung von Pansenbakterien dient. Mit den erfindungsgemäßen Beschichtungen wird nach 6 h nach der oralen Verabreichung in der Regel eine mind. 2 gew.-%ige, vorzugsweise 5 gew.-%ige und insbesondere eine 10 gew.-%ige Freisetzung der biologisch wirksamen Substanz erreicht. In dieser Hinsicht ist die erfindungsgemäße Beschichtung den rein pH-abhängigen Beschichtungen deutlich überlegen, da diese in Pansen in der Regel gar keinen Wirkstoff, im Magen hingegen eine schlagartige Freisetzung des Wirkstoffes bewirken.

Für andere Anwendungen als bei Wiederkäuern wird die Freisetzung des Wirkstoffes in der Regel so eingestellt, daß nach 2 h nach der oralen Einnahme höchstens 50 Gew.-%, vorzugsweise höchstens 30 Gew.-% und insbesondere höchstens 20 Gew.-% freigesetzt sind. Hierbei sollten dann nach 8 h mind. 40 Gew.-%, vorzugsweise mind. 50 Gew.-% und besonders bevorzugt mind. 75 Gew.-% freigesetzt sein. Solche Werte erlauben eine im wesentlichen gleichmäßige Versorgung des Organismus mit dem Wirkstoff bei nur zweimaliger Verabreichung am Tag. Die mit den erfindungsgemäßen dünneren Bereichen und/oder Sollbruchstellen ausgerüsteten Präparate geben dabei in der Regel mind. 20 % mehr Wirkstoff nach den o. g. Zeiten ab, als die Präparate mit einer im wesentlichen gleichmäßig dicken Beschichtung, meist werden sogar mehr als 30 und in besonderen Fällen mehr als 50 Gew.-% freigesetzt.

Die erfindungsgemäße Beschichtung hat dabei den Vorteil, daß je nach Zusammensetzung und/oder Aufbau der Beschichtung deren Eigenschaften so eingestellt werden können, daß die Freisetzung des Wirkstoffes weitgehend unabhängig vom pH-Wert des jeweiligen Mediums oder des Vorhandenseins von Enzymen oder sonstigen zerfallsfördernden Stoffen vorteilhaft ganz oder teilweise in den Bereichen des Gastrointestinaltraktes erfolgt, in denen die Verfügbarkeit des Wirkstoffes erwünscht ist. Beispielsweise kann erreicht werden, daß ein geringer Teil des Wirkstoffes bereits im Pansen frei wird, wie dies etwa im Falle des Nicotinamids erwünscht ist, und/oder, daß die Hauptmenge des Wirkstoffes schnell oder in einigen Fällen mit slow-release-Effekt im Dünndarm, dem eigentlichen Resorptionsort für den Wirkstoff, zur Verfügung steht.

Besonders wichtig bei der erfindungsgemäßen Wirkstoffzubereitung ist, daß die Celluloseether und die Füllstoffe bereits seit langem mit Erfolg in der Tierernährung eingesetzt werden und uneingeschränkt nach dem Futtermittelrecht zugelassen sind.

Die Bezeichung Wirkstoff bezieht sich im vorliegenden Zusammenhang auf Tierfutter, Nahrungsmittel, Nährstoffe und Arzneimittel. Namentlich sind dies zum Beispiel Proteine, Aminosäuren und Aminosäuredieri-

4

vate, Vitamine, Kohlenhydrate, Hormone und andere (Tier)arzneimittel. Beispiele für Proteine sind etwa Federmehl, Fischmehl, Casein oder Kartoffeleiweiß; für Aminosäuren und Aminosäurederivate Methionin, Lysin, Threonin, Tryptophan, N-Acylaminosäuren, Hydroxyaminosäuren oder deren physiologisch verträglichen (Metall)salze oder Peptide; für Vitamine Vitamin A, Vitamin A-Acetat, Vitamin D, Vitamin E, Nicotinsäure oder Nicotinsäureamid, die B-Vitamine oder Cholinchlorid; für Kohlenhydrate Glucose, Stärke oder Saccharose; für Hormone und (Tier)arzneimittel Östrogen, Tyrotropin, Antibiotika, Anthelmintika oder Antiparasitika. Selbstverständlich können auch Kombinationen mehrerer derartiger Wirkstoffe eingesetzt werden.

Der Wirkstoff oder die Wirkstoffkombination wird, vorteilhaft unter Mitverwendung eines Bindemittels, unter Einsatz gängiger Kompaktierungsmethoden, wie Extrusion, Tablettierung, Sprüh- , Fließbett- oder Rührgranulation zu Pellets, Tabletten oder Granulaten geformt und verdichtet. Als Bindemittel können Stoffe, wie nicht-toxische Gummis, Stärke, Gelatine, Cellulosederivate, Alginate und ähnliche an sich bekannte Substanzen zugesetzt werden, die üblicherweise in der Lebensmittel- oder Futterverarbeitung als Binde-, Gelier-, Verdickungs- oder Tablettierungsmittel Verwendung finden.

Gegebenenfalls können weitere Stoffe, wie Kieselsäuren, Silikate, Metallcarbonate, -phosphate oder -oxide und Alkalimetallstearate als Fließhilfsmittel, Gleitmittel, Dichteregulatoren oder Adsorbentien für flüssige Wirkstoffe zugesetzt werden.

Die so hergestellten Wirkstoffkerne werden anschließend derart mit der schützenden Beschichtung umhüllt, daß sie mit einer Lösung des Filmbildners, in der gegebenenfalls der Füllstoff suspendiert ist, in Kontakt gebracht werden und dann das Lösungsmittel verdampft wird. Geeignete Lösungsmittel finden sich unter den Kohlenwasserstoffen, kurzkettigen Alkoholen oder Ketonen und sind zum Beispiel Toluol, Isopropylalkohol, Methanol, Ethanol, Aceton oder Gemische derartiger Lösungsmittel.

Der darin lösliche Filmbildner ist vorteilhaft da zugelassen ein Celluloseether, der vorteilhafterweise in Wasser nicht oder schwer löslich ist, oder ein Gemisch von mehreren solchen Celluloseethern, vorzugsweise aber Ethylcellulose.

Der Füllstoff wird vorteilhaft ausgewählt unter den oben genannten Stoffen. Selbstverständlich können auch Gemische derartiger Füllstoffe eingesetzt werden. Der Füllstoff hat mehrere günstige Effekte: Einerseits versprödet er den Filmbildner und erleichtert hierdurch das Aufbrechen der Beschichtung, andererseits wirkt er wie ein Schwamm für die Magensäfte (o. a.) und erleichtert hierdurch das Ab- oder Auflösen der Beschichtung, und schließlich erlaubt die Füllstoffschicht das Aufbringen einer dünneren äußeren Schicht, so daß insbesondere das oben beschriebene mechanische Ablösen der Beschichtung erleichtert wird. Eine Beschichtung durch und durch mit hohem Füllstoffgehalt ist meist nicht geeignet, da diese sich zu schnell auflöst.

Als Beschichtungsverfahren eignen sich die an sich bekannten und üblichen Methoden, zum Beispiel verschiedene Wirbelbett- und Dragierverfahren.

Die Herstellung der erfindungsgemäßen Wirkstoffzubereitung erfolgt in einem typischen Fall so, daß der Wirkstoff mit 5 bis 95 Gew.-%, vorzugsweise 10 bis 20 Gew.-%, bezogen auf das Gewicht des Wirkstoffes, an dem Bindemittel oder einem Gemisch aus dem Bindemittel und Wasser oder einer gesättigten Lösung des Wirkstoffes in Wasser vermengt und zu Tabletten oder Pellets verpreßt wird, oder daß der Wirkstoff zusammen mit dem Bindemittel granuliert wird. Die Tabletten oder Pellets sollen vorzugsweise 0.5 bis 2 mm x 1 bis 5 mm und die (kantigen) Granulate 1 bis 2 mm groß sein, können aber auch größer oder kleiner sein. Bei dieser Beschichtung sind besonders Pellets und Tabletten bevorzugt, da mit diesen auf einfache Weise auch die dünnen Bereiche oder Sollbruchstellen erhalten werden.

Nach dem Trocknen werden die so hergestellten Wirkstoffkerne zuerst mit der Suspension des Füllstoffes in einer Lösung des Filmbildners und dann mit der reinen Lösung des Filmbildners besprüht. Die Beschichtung macht, bezogen auf das Gewicht des Wirkstoffkerns, 1,5 bis 30 Gew.-%, vorzugsweise 2,5 bis 20 Gew.-% und insbesondere 4 bis 14 Gew.-% aus und enthält, wiederum bezogen auf das Gewicht des Wirkstoffkerns, 1 bis 20 Gew.-%, vorzugsweise 2 bis 15 Gew.-% und insbesondere 3 bis 10 Gew.-% Filmbildner und 0,5 bis 10 Gew.-%, vorzugsweise 0,5 bis 5 Gew.-% und insbesondere 1 bis 4 Gew.-% Füllstoff.

Die Beschichtung wird im allgemeinen so aufgebracht, daß eine 2 bis 20 Gew.-%ige, vorzugsweise 4 bis 15 Gew.-%ige Lösung des Filmbildners in einem geeigneten Lösungsmittel oder Lösungsmittelgemisch hergestellt und so geteilt und nacheinander aufgesprüht wird, daß die innere Schicht, wiederum bezogen auf das Gewicht des Wirkstoffkerns, 0,2 bis 8 Gew.-%, vorzugsweise 0,2 bis 5 Gew.-% und insbesondere 0,5 bis 3,5 Gew.-% Filmbilner und die Gesamtmenge an Füllstoff enthält. Nach dem Verdampfen des oder der Lösungsmittel(s) aus dieser ersten Schicht wird der Rest der Filmbildner-Lösung zur Bildung einer zweiten Schicht aufgesprüht und es werden wieder das beziehungsweise die Lösungsmittel verdampft.

Werden kantige Wirkstoffkerne wie z. B. Pellets oder Tabletten auf diese Weise beschichtet, so erhält man eine Beschichtung, die an den Kanten deutlich dünner ist. An diesen "Schwachstellen" kann dann das Teilchen im Magen oder Darm aufbrechen, selbst wenn in den Verdauungssäften nur schlecht oder gar nicht lösliche Filmbildner, wie z. B. z. T. obige Celluloseether, verwendet werden. Selbstverständlich soll der Beschichtungsvorgang vorsichtig erfolgen, damit die Kanten nicht zu sehr verrundet werden.

Durch die nachfolgenden Beispiele und Vergleichsversuche soll die Erfindung näher veranschaulicht werden. Alle Prozentangaben bedeuten Gew.-%e.

Zur Abschätzung der Schutzwirkung der Beschichtung kann vorteilhaft ein in-vitro-Test zugrundegelegt werden, der zumindest bezüglich des pH-Wertes, der Temperatur und teilweise auch hinsichtlich der Bewegung die Bedingungen im Verdauungssystem z. B. eines Wiederkäuers simuliert. Dazu wird die hergestellte Wirkstoffzubereitung in einem Schüttelwasserbad bei 37°C nacheinander in drei verschiedenen Pufferlösungen inkubiert, und zwar zunächst 6 Stunden lang in einem Citratpuffer, hergestellt durch Auflösen von 72 g Citronensäure x 1 $H_2O$ und 19,5 g NaOH in Wasser und Verdünnen mit Wasser auf 1 Liter, mit einem pH-Wert von 5,5; anschließend 2 Stunden lang in einem HCl/KCl-Puffer, hergestellt durch Verdünnen von 250 ml 0,2 M KCl-Lösung und 65 ml 0,2 M HCl-Lösung mit Wasser auf 1 Liter, mit einem pH-Wert von 2,0; und schließlich 16 Stunden lang in einem Citratpuffer, hergestellt durch Auflösen von 42 g Citronensäure x 1 $H_2O$ und 23 g NaOH in Wasser und Verdünnen mit Wasser auf 1 Liter, mit einem pH-Wert von 6,5. Jeweils nach der angegebenen Zeit wird der ungelöste Anteil des Wirkstoffes in den Teilchen durch HPLC bestimmt und bezogen auf 100 prozentigen Gehalt vor dem Test in der Form a % nach der Inkubation bei pH 5,5 / b % nach der Inkubation bei pH 2,0 / c % nach der Inkubation bei pH 6,5 angegeben.

Es hat sich herausgestellt, daß die so erhaltenen Werte tendenziell gut mit Ergebnissen von in-vivo-Versuchen korrelieren und unter vorläufigem Verzicht auf Tierversuche eine rasche und verläßliche Aussage über die Eignung der getesteten Teilchen liefern.

Beispiel 1:

3 600 g DL-Methionin und 400 g des Natriumsalzes von Carboxymethylcellulose werden vorgelegt und unter intensivem Mischen mit 870 g Wasser versetzt. Die Mischung wird in einer Ringkollerpresse mit 1,5 mm Matrizenbohrung zu Pellets verpreßt, die auf eine Länge von ca. 2 mm abgeschnitten und bei 60°C getrocknet werden.

100 g dieser Pellets werden in einer Dragiervorrichtung zunächst mit einer Lösung von 0,5 g Ethylcellulose in 25 ml Ethanol, in der 2,5 g Natriumaluminiumsilikat (Teilchengröße 3,5 μm) suspendiert sind, besprüht. Anschließend wird eine Lösung von 3,5 g Ethylcellulose in 95 ml Ethanol aufgesprüht. Die so beschichteten Teilchen werden unter vermindertem Druck bei 60°C getrocknet. Ihr durch bromatometrische Titration bestimmter Gehalt an Methionin beträgt 83,6 % und der jeweils ungelöste Anteil des Methionins 75 % / 63 % / 18 %.

Beispiel 2:

100 g der nach Beispiel 1 hergestellten Pellets werden wie im Beispiel 1 unter Verwendung von insgesamt 4 g Ethylcellulose (innen 1 g, außen 3 g) und 1,5 g Natriumaluminiumsilikat beschichtet. Der Gehalt an Methionin beträgt 84,6 % und der jeweils ungelöste Anteil des Methionins 70 % / 56 % / 13 %.

Beispiel 3:

100 g der nach Beispiel 1 hergestellten Pellets werden wie im Beispiel 1 unter Verwendung von insgesamt 5 g Ethylcellulose (innen 1,25 g, außen 3,75 g) und 2,5 g Natriumaluminiumsilikat beschichtet. Der Gehalt an Methionin beträgt 83,0 % und der jeweils ungelöste Anteil an Methionin 77 % / 64 % / 21 %.

Beispiel 4:

100 g der nach Beispiel 1 hergestellten Pellets werden wie im Beispiel 1 unter Verwendung von insgesamt 8 g Ethylcellulose (innen 2 g, außen 6 g) und 5 g Natriumaluminiumsilikat beschichtet. Der Gehalt an Methionin beträgt 79,5 % und der jeweils ungelöste Anteil an Methionin 93 % / 87 % / 47 %.

Vergleichsbeispiel 5:

Die nach Beispiel 1 hergestellten Pellets werden in einer dazu geeigneten und üblichen Apparatur, z. B. einem Rondiergerät, so behandelt, daß die erfindungsgemäßen Kanten und Stege weitgehend abgeschliffen

6

EP 0 495 349 B1

werden. Anschließend werden 100 g der so gerundeten Pellets wie im Beispiel 1 unter Verwendung von insgesamt 4 g Ethylcellulose (innen 0,5 g, außen 3,5 g) und 2 g Natriumaluminiumsilikat beschichtet. Der Gehalt an Methionin beträgt 84,8 % und der jeweils ungelöste Anteil an Methionin 94 % / 91 % / 74 %.

Dieser Vergleichsversuch zeigt, daß nach Abschleifen der Kanten und Stege und damit Wegfall von Sollbruchstellen der weitaus größte Teil des Methionins auch nach 24 h noch ungelöst bleibt.

Beispiel 6:

100 g der nach Beispiel 1 hergestellten Pellets werden wie im Beispiel 1 unter Verwendung von insgesamt 5 g Ethylcellulose (innen 1,25 g, außen 3,75 g) und von 2,5 g Ammoniumalginat als Füllstoff beschichtet. Der Gehalt an Methionin beträgt 84,1 % und der jeweils ungelöste Anteil des Methionins 86 % / 76 % / 34 %.

Beispiel 7:

100 g der nach Beispiel 1 hergestellten Pellets werden wie im Beispiel 1 unter Verwendung von insgesamt 5 g Ethylcellulose (innen 1,25 g, außen 3,75 g) und von 2,5 g Magnesiumcarbonat als Füllstoff beschichtet. Der Gehalt an Methionin beträgt 83,4 % und der jeweils ungelöste Anteil des Methionins 91 % / 82 % / 43 %.

Beispiel 8:

100 g der nach Beispiel 1 hergestellten Pellets werden wie im Beispiel 1 unter Verwendung von insgesamt 5 g Ethylcellulose (innen 1,25 g, außen 3,75 g) und von 2,5 g Stärke als Füllstoff beschichtet. Der Gehalt an Methionin beträgt 83,4 % und der jeweils ungelöste Anteil des Methionins 84 % / 75 % / 31 %.

Beispiel 9:

100 g der nach Beispiel 1 hergestellten Pellets werden wie im Beispiel 1 unter Verwendung von insgesamt 5 g Ethylcellulose (innen 1,25 g, außen 3,75 g) und von 2,5 g Gummi arabicum als Füllstoff beschichtet. Der Gehalt an Methionin beträgt 83,8 % und der jeweils ungelöste Anteil des Methionins 91 % / 83 % / 40 %.

Beispiel 10:

100 g der nach Beispiel 1 hergestellten Pellets werden wie im Beispiel 1 unter Verwendung von insgesamt 5 g Ethylcellulose (innen 1,25 g, außen 3,75 g) und von 2,5 g Natriumstearat als Füllstoff beschichtet. Der Gehalt an Methionin beträgt 82,9 % und der jeweils ungelöste Anteil des Methionins 78 % / 65 % / 18 %.

Beispiel 11:

Es wird verfahren wie im Beispiel 1, aber bei der Herstellung der Wirkstoffpellets werden anstelle des Natriumsalzes von Carboxymethylcellulose 400 g Stärke verwendet.

100 g dieser Pellets werden wie in Beispiel 1 unter Verwendung von insgesamt 5 g Ethylcellulose (innen 1,25 g, außen 3,75 g) und von 2,5 g Natriumaluminiumsilikat als Füllstoff beschichtet. Der Gehalt an Methionin beträgt 84,2 % und der jeweils ungelöste Anteil des Methionins 80 % /69 % / 29 %.

Beispiel 12:

Es wird verfahren wie im Beispiel 1, aber bei der Herstellung der Wirkstoffpellets werden anstelle des Natriumsalzes von Carboxymethylcellulose 360 g Stärke und 40 g Natriumstearat verwendet.

100 g dieser Pellets werden unter Verwendung von insgesamt 3,5 g Ethylcellulose (innen 1 g, außen 2,5 g) und von 2 g Natriumaluminiumsilikat als Füllstoff beschichtet. Der Gehalt an Methionin beträgt 85,5 % und der jeweils ungelöste Anteil des Methionins 66 % / 54 % / 8 %.

7

Beispiel 13:

100 g der nach Beispiel 12 hergestellten Pellets werden unter Verwendung von insgesamt 4 g Ethylcellulose (innen 1 g, außen 3 g) und von 2 g Natriumstearat als Füllstoff beschichtet. Der Gehalt an Methionin beträgt 84,8 % und der jeweils ungelöste Anteil des Methionins 73 % / 63 % / 19 %.

Vergleichsbeispiel 14:

100 g der nach Beispiel 1 hergestellten Pellets werden in nur einer Schicht mit einer Lösung von 7,5 g Ethylcellulose in 200 ml Ethanol ohne Verwendung eines Füllstoffes beschichtet. Der Gehalt an Methionin beträgt 83,4 % und der jeweils ungelöste Anteil des Methionins 88 % / 81 % / 54 %.

Dieser Vergleichsversuch zeigt, daß bei einer relativ dicken und elastischen Beschichtung des Wirkstoffkerns in einer Schicht der größere Teil des Methionins auch nach insgesamt 24 Stunden ungelöst bleibt.

Beispiel 15:

100 g der nach Beispiel 1 hergestellten Pellets werden in nur 1 Schicht mit einer Lösung von 5 g Ethylcellulose in 400 ml Ethanol, in der 2,5 g Natriumaluminiumsilikat suspendiert sind, beschichtet. Der Gehalt an Methionin beträgt 83,1 % und der jeweils ungelöste Anteil des Methionins 36 % / 19 % / 0 %.

Dieser Versuch zeigt im Vergleich mit Beispiel 3, daß die Beschichtung mit zwar der gleichen Menge an Ethylcellulose und Natriumaluminiumsilikat, aber nur in einer Schicht schon unter den Bedingungen im Pansen das Methionin nur unzureichend vor Auflösung schützt.

Hingegen ist diese Beschichtung geeignet für Nicht-Wiederkäuer, da nach 8 h erst 81 % des Wirkstoffes freigesetzt sind. Der erhaltene slow-release-Effekt bewirkt bei 2 Dosisgaben/Tag eine gleichmäßige Versorgung des Organismus mit dem Wirkstoff.

Tierversuche:

Die folgenden Untersuchungen zeigen anhand von Tierversuchen die überlegene Wirkung des erfindungsgemäßen Produktes. Untersucht wurde der Anstieg des Methioningehalts im Blutplasma von Milchkühen. Da alle Nährstoffe, die in der Milch enthalten sind, bzw. deren Vorstufen mit dem Blutstrom der Milchdrüse zugeführt werden, muß das Methionin aus einem geschützten Produkt zunächst dort im Blut ankommen.

Vorversuch:

In Voruntersuchungen, in denen Methioninpräparate nach 6stündiger Vorinkubation im Pansen (Simulation der natürlichen Verweildauer) direkt in den Labmagen gegeben wurden, wird das Ausmaß des Methioninanstieges im Blutplasma bei Verabreichung definierter Mengen an geschütztem Methionin geprüft.

Das zu prüfende Produkt wurde in Portionen von 25 g in Nylonbeutel (30 μm Porengröße) eingenäht und über 6 h im Pansen der Versuchskühe (3 Tiere) vorinkubiert (max. 6 - 8 Beutel pro Tier). Nach der Entnahme wurden die Beutel von Futterpartikeln gereinigt, aber nicht gewaschen. Die Inhalte aller Beutel wurden gepoolt und zur Ermittlung des Substanzverlustes gewogen. In einer Probe des vorinkubierten gepoolten Materials wurde der Methioningehalt bestimmt, woraus sich die während der 6 h verschwundene Methioninmenge zu 17 Gew.-% bzw. die Menge an pansenstabilem Methionin zu 83 Gew.-% errechnen läßt. Ein ebenfalls getestetes bekanntes Produkt (Vergleichsbeispiel 17) war im Pansen zu 100 % stabil.

Das vorinkubierte Material wurde anschließend in Gelantinekapseln eingewogen, die sich innerhalb weniger Minuten im Labmagen auflösen. Die Verabreichung erfolgte bei den 3 Tieren viermal täglich durch die Pansenfistel direkt in den Labmagen. Über einen Zeitraum von vier Tagen (Tag 1 - 4) wurden pro Tag 25 g Methionin in dieser Form verabreicht.

Tag 0 diente der Gewinnung der Kontrollparameter. An den Tagen 0, 3 und 4 wurden bei den Tieren Blutproben durch Punktion der Vena jugularis jeweils um 13 h und 16 h gezogen. In diesen Proben wurde der Gehalt an freiem Methionin im Pansen bestimmt. Es zeigte sich ein Anstieg des Plasmamethioninspiegels von etwa 100 % im Mittel der Tage 3 und 4 im Vergleich zu Tag 0.

Beispiel 16 und Vergleichsbeispiel 17:

Im Versuch erhielten die Tiere (jeweils 4 pro Produkt) zu einer bedarfsdeckenden Ration, bestehend aus 10 % Heu, 30 % Grassilage, 20 % Maissilage (zusammen 15 kg Trockenmasse/Tag) und 40 % eines Getreide/Sojaschrot-Kraftfutters (6 kg Trockenmasse/Tag), 30,6 g DL-Methionin in Form von nach Beispiel 12 hergestellten Pellets, die unter Verwendung von insgesamt 4 Gew.-% Ethylcellulose bezogen auf das Gewicht des Wirkstoffkerns (innen 0,5 %, außen 3,5 %) und von 2 Gew.-% Natriumaluminiumsilikat als Füllstoff beschichtet wurden (Beispiel 16) bzw. 25 g in Form von Wirkstoffgranulaten, die 50 Gew.-% DL-Methionin enthalten und die ihren Schutz aus einer Beschichtung mit Monocarbonsäuren mit 14 - 22 C-Atomen beziehen (Vergleichsbeispiel 17).

Solche Produkte sind aus der EP 0 037 478 und DE-PS 22 12 568 bekannt und käuflich erwerbbar.

Die Mengen waren so berechnet, daß aufgrund der im Vorversuch gemessenen Pansenstabilität der Produkte etwa 25 g DL-MET in den Dünndarm gelangen sollten (83 % Stabilität für das Beispiel 16 und 100 % für den Vergleich 17). Die Präparate wurden 12 Tage lang verabreicht (Tag 1 - 12). An den Tagen 0, 10, 12 und 14 wurden Blutproben gezogen (Vena jugularis, 11.50 und 14.50 Uhr). Die Ergebnisse der Messungen zeigt die Tabelle.

In beiden Fällen steigt der Methioningehalt im Blutplasma an, wobei der Anstieg beim Beispiel 16 mit 188 % an Tag 10 im Vergleich zum Vergleich 17 mit 28 % deutlich höher ist. An Tag 12, dem letzten Tag der oralen Gabe, sind die entsprechenden Werte +151 % und +5 %. Zwei Tage nach dem Absetzen ist der Methioningehalt im Blutplasma wieder auf das Ausgangsniveau zurückgegangen, in der Vergleichsbehandlung liegt er sogar 10 % unter dem Ausgangswert.

Dementsprechend sind die Unterschiede im Methioningehalt im Kot. Während im Mittel der Tage 10 und 12 über die vier Kühe beim Beispiel 16 0,074 % Methionin im Kot gefunden werden, sind es beim Vergleich 17 0,246 %.

Tabelle : Der Einfluß von geschützten Methioninprodukten auf die Plasmamethioninspiegel von Kühen nach Verfütterung mit Kraftfutter (KF) (Mittel aus je 4 Kühen und 2 Uhrzeiten)

| Beispiel | tägl. MET-Gabe über das KF (g) | tägl. MET (g) am Dünndarm (errechnet aus Vorversuch) | Blut-MET-Spiegel (µmol/l) Kontr. kein MET je zwei Werte | | | | Anstieg des Blut-MET-Spiegels (rel. Zunahme in %) | | |
|---|---|---|---|---|---|---|---|---|---|
| | | | Tag 0 | Tag 10 | Tag 12 | Tag 14 | Tag 10 | Tag 12 | Tag 14 |
| 16 | 30,6 | 25,4 | 8,3 ± 4,0 | 23,8 ± 8,8 | 20,8 ± 6,0 | 8,4 ± 5,8 | 188 | 151 | 1 |
| Vergleich 17 | 25,0 | 25,0 | 13,4 ± 4,2 | 17,2 ± 4,8 | 14,1 ± 5,9 | 12,1 ± 4,5 | 28 | 5 | - 10 |

**Patentansprüche**

1. Wirkstoffzubereitung zur oralen Verabreichung insbesondere an Wiederkäuer, enthaltend einen mindestens eine biologisch wirksame Substanz enthaltenden Wirkstoffkern und eine diesen Kern umhüllenden Beschichtung, die eine verzögerte Freisetzung des Kerns nach der oralen Verabreichung bewirkt, **dadurch gekennzeichnet**, daß die umhüllende Beschichtung dünnere Bereiche und Sollbruchstellen aufweist, die die verzögerte Freisetzung gegenüber einer im wesentlichen gleichmäßigen Beschichtung beschleunigen.

2. Wirkstoffzubereitung nach Anspruch 1, **dadurch gekennzeichnet**, daß die Umhüllung bei Körpertemperatur spröde ist.

3. Wirkstoffzubereitung nach Anspruch 1 oder 2, **dadurch gekennzeichnet**, daß der Wirkstoffkern eine pharmazeutisch wirksame Substanz oder ein Nährmittel enthält.

4. Wirkstoffzubereitung nach einem der vorhergehenden Ansprüche, **dadurch gekennzeichnet**, daß die dünneren Bereiche und Sollbruchstellen eine in sich geschlossene Linie bilden.

5. Wirkstoffzubereitung nach Anspruch 4, **dadurch gekennzeichnet**, daß die Linie im wesentlichen kreisförmig ist.

6. Wirkstoffzubereitung nach Anspruch 5, **dadurch gekennzeichnet**, daß mindestens zwei solcher kreisförmiger Linien vorgesehen sind.

7. Wirkstoffzubereitung nach einem der vorhergehenden Ansprüche, **dadurch gekennzeichnet**, daß sich die dünneren Bereiche über mindestens 0,5 % der Gesamtfläche der Beschichtung erstrekken.

8. Wirkstoffzubereitung nach Anspruch 7, **dadurch gekennzeichnet**, daß die dünneren Bereiche mindestens 2 % der Gesamtfläche der Beschichtung ausmachen.

9. Wirkstoffzubereitung nach einem der vorhergehenden Ansprüche, **dadurch gekennzeichnet**, daß die dünneren Bereiche mindestens 20 % unter der mittleren Schichtdicke der umhüllenden Beschichtung liegen.

10. Wirkstoffzubereitung nach Anspruch 9, **dadurch gekennzeichnet**, daß die dünneren Bereiche mindestens 30 % dünner sind als die mittlere Schichtdicke der Beschichtung.

11. Wirkstoffzubereitung nach einem der vorhergehenden Ansprüche, **dadurch gekennzeichnet**, daß die dünneren Bereiche und/oder Sollbruchstellen durch Kanten gebildet werden.

12. Wirkstoffzubereitung nach einem der vorhergehenden Ansprüche, **dadurch gekennzeichnet**, daß die Kanten zwischen Flächen liegen, die in einem Winkel von ≤ 120°, vorzugsweise ≤ 90° und insbesondere ≤ 70° zueinander stehen.

13. Wirkstoffzubereitung nach Anspruch 12, **dadurch gekennzeichnet**, daß die Flächen senkrecht zur Kante eine Länge von mindestens 0,05 mm, vorzugsweise mindestens 0,1 mm und insbesondere mindestens 0,2 mm aufweisen.

**14.** Wirkstoffzubereitung nach einem der Ansprüche 11 bis 13,
**dadurch gekennzeichnet**,
daß die Kanten einen Radius von ≤ 1 mm, vorzugsweise ≤ 0,7 mm und insbesondere ≤ 0,5 mm aufweisen.

**15.** Wirkstoffzubereitung nach Anspruch 14,
**dadurch gekennzeichnet**,
daß der Radius höchstens die Länge der an die Kante angrenzenden Flächen senkrecht zur Kante hat.

**16.** Wirkstoffzubereitung nach Anspruch 15,
**dadurch gekennzeichnet**,
daß der Radius höchstens 2/3 und insbesondere höchstens 1/2 der Lange beträgt.

**17.** Wirkstoffzubereitung nach einem der vorhergehenden Ansprüche,
**dadurch gekennzeichnet**,
daß die Freisetzung der wirksamen Substanz so bemessen ist, daß nach 6 h nach der oralen Verabreichung höchstens 50 %, vorzugsweise höchstens 30 % und insbesondere höchstens 20 % freigesetzt sind.

**18.** Wirkstoffzubereitung nach Anspruch 17,
**dadurch gekennzeichnet**,
daß nach 6 h nach der oralen Verabreichung mindestens 2 Gew.-%, vorzugsweise 5 Gew.-% und insbesondere 10 Gew.-% der biologisch wirksamen Substanz freigesetzt sind.

**19.** Wirkstoffzubereitung nach einem der vorhergehenden Ansprüche,
**dadurch gekennzeichnet**,
daß nach 24 h nach der oralen Verabreichung mindestens 50 Gew.-%, vorzugsweise mindestens 70 Gew.-% und insbesondere mindestens 80 Gew.-% der biologisch wirksamen Substanz freigesetzt sind.

**20.** Wirkstoffzubereitung zur oralen Verabreichung insbesondere an Wiederkäuer nach einem der vorhergehenden Ansprüche,
**dadurch gekennzeichnet**,
daß die Beschichtung 1,5 bis 30 Gew.-%, bezogen auf das Gewicht des Wirkstoffkerns, ausmacht und, jeweils bezogen wiederum auf das Gewicht des Wirkstoffkerns, 1 bis 20 Gew.-% eines Celluloseethers als Filmbildner und 0,5 bis 10 Gew.-% mindestens eines Füllstoffes, ausgewählt unter Metallcarbonaten, Kieselsäuren, Silikaten, Alginaten, Stearaten, Stärken und Gummis, enthält mit der Maßgabe, daß die Beschichtung in zwei Schichten aufgetragen ist, deren innere, kernnahe Schicht, bezogen wiederum auf das Gewicht des Wirkstoffkerns, 0,2 bis 8 Gew.-% Filmbildner und die Gesamtmenge an Füllstoff und die äußere Schicht die Restmenge des Filmbildners enthält.

**21.** Wirkstoffzubereitung nach Anspruch 20,
**dadurch gekennzeichnet**,
daß der Filmbildner Ethylcellulose ist.

**22.** Wirkstoffzubereitung nach Anspruch 20 oder 21,
**dadurch gekennzeichnet**,
daß der Füllstoff Magnesium-, Calcium- oder Natriumcarbonat, eine Fällungskieselsäure, ein Calcium-, Aluminium- oder Natriumaluminiumsilikat, Calcium-, Natrium- oder Ammoniumalginat, Natriumstearat, Maisstärke oder Gummi arabicum oder ein Gemisch aus zwei oder mehr dieser Stoffe ist.

**23.** Verwendung einer Wirkstoffzubereitung nach einem der vorhergehenden Ansprüche mit einem Nährmittel als Kern in einem Wiederkäuerfutter oder einer sonstigen Zusammensetzung zur Ernährung von Wiederkäuern.

**24.** Verfahren zur Versorgung von Wiederkäuern mit Nährstoffen
**dadurch gekennzeichnet**,
daß man den Wiederkäuern eine Wirkstoffzubereitung nach einem der vorhergehenden Ansprüche mit einem Nährmittel als Kern oral verabreicht.

**Claims**

1.   An active-substance preparation for oral administration, more particularly to ruminants, containing an active-substance core containing at least one biologically active substance and a coating around this core which delays the release of the core after the oral administration, characterized in that the surrounding coating has relatively thin zones and weak points which accelerate the delayed release in relation to a substantially uniform coating.

2.   An active-substance preparation as claimed in claim 1, characterized in that the coating is brittle at body temperature.

3.   An active-substance preparation as claimed in claim 1 or 2, characterized in that the active-substance core contains a pharmaceutically active substance or a nutrient.

4.   An active-substance preparation as claimed in any of the preceding claims, characterized in that the relatively thin zones and weak points form a continuous line.

5.   An active-substance preparation as claimed in claim 4, characterized in that the line is substantially circular.

6.   An active-substance preparation as claimed in claim 5, characterized in that at least two such circular lines are provided.

7.   An active-substance preparation as claimed in any of the preceding claims, characterized in that the relatively thin zones extend over at least 0.5% of the total surface area of the coating.

8.   An active-substance preparation as claimed in claim 7, characterized in that the relatively thin zones make up at least 2% of the total surface area of the coating.

9.   An active-substance preparation as claimed in any of the preceding claims, characterized in that the relatively thin zones are at least 20% thinner than the average layer thickness of the surrounding coating.

10.  An active-substance preparation as claimed in claim 9, characterized in that the relatively thin zones are at least 30% thinner than the average layer thickness of the coating.

11.  An active-substance preparation as claimed in any of the preceding claims, characterized in that the relatively thin zones and/or weak points are formed by edges.

12.  An active-substance preparation as claimed in any of the preceding claims, characterized in that the edges are situated between surfaces which are at an angle of $\leq 120°$, preferably $\leq 90°$ and more preferably $\leq 70°$ to one another.

13.  An active-substance preparation as claimed in claim 12, characterized in that the surfaces have a length perpendicularly of the edge of at least 0.05 mm, preferably at least 0.1 mm and more preferably at least 0.2 mm.

14.  An active-substance preparation as claimed in any of claims 11 to 13, characterized in that the edges have a radius of $\leq 1$ mm, preferably $\leq 0.7$ mm and more preferably $\leq 0.5$ mm.

15.  An active-substance preparation as claimed in claim 14, characterized in that the radius has at most the length of the surfaces adjoining the edge perpendicularly thereof.

16.  An active-substance preparation as claimed in claim 15, characterized in that the radius is at most two thirds and, more particularly, at most one half the length.

17.  An active-substance preparation as claimed in any of the preceding claims, characterized in that the release of the active substance is measured in such a way that at most 50%, preferably at most 30% and more preferably at most 20% of the active substance is released 6 hours after its oral administration.

18. An active-substance preparation as claimed in claim 17, characterized in that at least 2% by weight, preferably 5% by weight and more preferably 10% by weight of the biologically active substance is released 6 hours after its oral administration.

19. An active-substance preparation as claimed in any of the preceding claims, characterized in that at least 50% by weight, preferably at least 70% by weight and more preferably at least 80% by weight of the biologically active substance is released 24 hours after its oral administration.

20. An active-substance preparation for oral administration, more particularly to ruminants, as claimed in any of the preceding claims, characterized in that the coating makes up 1.5 to 30% by weight, based on the weight of the active-substance core, and contains - again based on the weight of the active-substance core - 1 to 20% by weight of a cellulose ether as film former and 0.5 to 10% by weight of at least one filler selected from metal carbonates, silicas, silicates, alginates, stearates, starches and gums, with the proviso that the coating is applied in two layers of which the inner layer nearer the core contains - again based on the weight of the active-substance core - 0.2 to 8% by weight of film former and the entire quantity of filler while the outer layer contains the rest of the film former.

21. An active-substance preparation as claimed in claim 20, characterized in that the film former is ethyl cellulose.

22. An active-substance preparation as claimed in claim 20 or 21, characterized in that the filler is magnesium, calcium or sodium carbonate, a precipitated silica, a calcium, aluminium or sodium aluminium silicate, calcium, sodium or ammonium alginate, sodium stearate, cornstarch or gum arabic or a mixture of two or more of these substances.

23. The use of the active-substance preparation claimed in any of the preceding claims containing a nutrient as core in a feed for ruminants or any other composition for the feeding of ruminants.

24. A process for supplying ruminants with nutrients, characterized in that the active-substance preparation claimed in any of the preceding claims containing a nutrient as core is orally administered to the ruminants.

**Revendications**

1. Préparation de principe actif pour l'administration orale, notamment à des ruminants, comprenant au moins une substance biologiquement efficace contenant un noyau de principe actif et un enrobage entourant ce noyau qui provoque une libération retardée du noyau après l'administration orale, caractérisée en ce que le revêtement enrobant présente des domaines plus minces et des zones de rupture théorique, qui accélèrent la libération retardée par rapport à un enrobage essentiellement uniforme.

2. Préparation de principe actif selon la revendication 1, caractérisée en ce que l'enrobage est fragile à la température du corps.

3. Préparation de principe actif selon la revendication 1 ou 2, caractérisée en ce que le noyau de principe actif contient une substance pharmaceutiquement efficace ou un produit nutritif.

4. Préparation de principe actif selon l'une des revendications précédentes, caractérisée en ce que les domaines plus minces et les zones de rupture théorique forment une ligne refermée sur elle-même.

5. Préparation de principe actif selon la revendication 4 , caractérisée en ce que la ligne est essentiellement circulaire.

6. Préparation de principe actif selon la revendication 5, caractérisée en ce qu'on prévoit au moins deux telles lignes circulaires.

**7.** Préparation de principe actif selon l'une des revendications précédentes, caractérisée en ce que les domaines plus minces s'étendent sur au moins 0,5 % de la surface totale du revêtement.

**8.** Préparation de principe actif selon la revendication 7, caractérisée en ce que les domaines plus minces représentent au moins 2 % de la surface totale du revêtement.

**9.** Préparation de principe actif selon l'une des revendications précédentes, caractérisée en ce que les domaines les plus minces se situent au moins 20 % en dessous de l'épaisseur de couche moyenne du revêtement enrobant.

**10.** Préparation de principe actif selon la revendication 9, caractérisée en ce que les domaines plus minces se situent au moins 30 % en dessous de l'épaisseur de couche moyenne du revêtement enrobant.

**11.** Préparation de principe actif selon l'une des revendications précédentes , caractérisée en ce que les domaines plus minces et/ou les zones de rupture théorique sont formés par des bords.

**12.** Préparation de principe actif selon l'une des revendications précédentes, caractérisée en ce que les bords se trouvent entre des surfaces qui font entre elles un angle $\leq 120°$, de préférence $\leq 90°$ et notamment $\leq 70°$.

**13.** Préparation de principe actif selon la revendication 12, caractérisée en ce que les surfaces perpendiculaires aux bords présentent une longueur d'au moins 0,05 mm, de préférence au moins 0,1 mm et notamment au moins 0,2 mm.

**14.** Préparation de principe actif selon l'une des revendications 11 à 13, caractérisée en ce que les bords présentent un rayon de $\leq 1$ mm, de préférence $\leq 0,7$ mm et notamment $\leq 0,5$ mm.

**15.** Préparation de principe actif selon la revendication 14, caractérisée en ce que le rayon a au plus la longueur des surfaces contiguës perpendiculaires au bord.

**16.** Préparation de principe actif selon la revendication 15, caractérisée en ce que le rayon représente au plus 2/3 et notamment au plus 1/2 de la longueur.

**17.** Préparation de principe actif selon l'une des revendications précédentes, caractérisée en ce que la libération de la substance active est définie en ce que 6 h après l'administration orale au plus 50 %, de préférence au plus 30 % est notamment au plus 20 % sont libérés.

**18.** Préparation de principe actif selon la revendication 17, caractérisée en ce que 6 h après l'administration orale, au moins 2 % en poids , de préférence 5 % en poids et notamment 10 % en poids de la substance biologiquement active sont libérés.

**19.** Préparation de principe actif selon l'une des revendications précédentes, caractérisée en ce que 24 h après l'administration orale, au moins 50 % en poids, de préférence au moins 70 % en poids et notamment au moins 80 % en poids de la substance biologiquement efficace sont libérés.

**20.** Préparation de principe actif pour l'administration, notamment à des ruminants, selon l'une des revendications précédentes, caractérisée en ce que l'enrobage qui contient 1,5 à 30 % en poids rapporté au poids du noyau de principe actif et, toujours rapporté au poids du noyau de principe actif, 1 à 20 % en poids d'un éther de cellulose comme agent filmogène et 0,5 à 10 % en poids au moins d'une charge choisie parmi les carbonates métalliques, les silices, les silicates, les algénates, les stéarates , les amidons et les gommes, est déposé en deux couches, dont la couche interne contiguë au noyau, toujours rapportée au poids du noyau de principe actif, contient 0,2 à 8 % en poids de produit filmogène et la totalité de la charge tandis que la couche externe contient le reste de l'agent filmogène.

**21.** Préparation de principe actif selon la revendication 20, caractérisée en ce que l'agent filmogène est de l'éthylcellulose.

**22.** Préparation de principe actif selon la revendication 20 ou 21, caractérisée en ce que la charge est du carbonate de magnésium, de calcium ou de sodium, une silice précipitée, un aluminosilicate de calcium, d'aluminium ou de sodium, un alginate de calcium, de sodium ou d'aluminium, du stéarate de sodium, de l'amidon de maïs ou de la gomme arabique, ou un mélange de deux ou plusieurs de ces substances.

**23.** Utilisation d'une préparation de principe actif selon l'une des revendications précédentes, avec un aliment comme noyau dans un aliment de ruminant ou une autre composition pour nourrir des ruminants.

**24.** Procédé d'alimentation de ruminant en substances nutritives, caractérisé en ce qu'on administre par voie orale aux ruminants une préparation de principe actif selon l'une des revendications précédentes avec un aliment comme noyau.